# EUROPÄISCHE PATENTANMELDUNG

(11) **EP 0 585 921 A1**
(43) Veröffentlichungstag der Anmeldung: **09.03.1994**
(21) Anmeldenummer: 93114065.1
(22) Anmeldetag: 02.09.1993
(51) Int. Cl.: A61B 10/00

(54) **Biopsie-Zange**

(30) Priorität: 02.09.1992 DE 9211834 U
(71) Anmelder: Lindner, Andreas, Dr., D-81679 München (DE); Geissler, Norbert, Dipl.-Ing., D-82343 Pöcking (DE)
(72) Erfinder: Lindner, Andreas, Dr., D-81679 München (DE); Geissler, Norbert, Dipl.-Ing., D-82343 Pöcking (DE)
(74) Vertreter: Eitle, Werner, Dipl.-Ing.

(57) **Zusammenfassung**

Die Erfindung betrifft eine Biopsie-Zange mit zwei an einer Zangenachse (2) zueinander schwenkbar gelagerten Zangenlöffeln (3), die mit ihren den Löffeln abgewandten rückwärtigen Armen (4) gelenkig mit dem einen Ende von laschenförmigen Schwenkgliedern (5) verbunden sind, welche mit ihrem jeweiligen anderen Ende an einer Seilhülse (6) angelenkt sind, die an dem vorderen Ende eines in einem Mantel (7) hin- und herbewegbaren Seiles (8) sitzt, wobei an dem vorderen Ende des Mantels (7) die Zangenachse (2) gelagert ist. Die zur gelenkigen Verbindung der rückwärtigen Arme (4) der Zangenlöffel (3) mit den Schwenkgliedern (5) dienenden Gelenkachsen (19) einerseits und die zur Verbindung der Schwenkglieder (5) mit der Seilhülse (6) dienenden weiteren Gelenkachsen (21) andererseits, sind als Achsstummel (19, 21) ausgebildet, welche jeweils einstückig mit einem der beiden gelenkig miteinander verbundenen Teile der Biopsie-Zange ausgebildet sind.

## Beschreibung

Die Erfindung betrifft eine Biopsie-Zange mit zwei an einer Zangenachse zueinander schwenkbar gelagerten Zangenlöffeln die mit ihren, den Löffeln abgewandten rückwärtigen Armen gelenkig mit dem einen Ende von laschenförmigen Schwenkgliedern verbunden sind, welche mit ihrem jeweiligen anderen Ende an einer Seilhülse angelenkt sind, die an dem vorderen Ende eines in einem Mantel hin- und herbewegbaren Seiles sitzt, wobei an dem vorderen Ende des Mantels die Zangenachse gelagert ist.

In einer bisher bekannten Biopsie-Zange sind die Achsen zur Verbindung der Zangenlöffel mit den laschenförmigen Schwenkgliedern und die Achsen zur Verbindung der Schwenkglieder mit der Seilhülse aus Nieten gebildet, die in Löcher der zu verbindenden Teile eingesetzt sind. Dies ist nicht nur hinsichtlich der erforderlichen Einzelteile, sondern auch aufgrund der zusätzlichen Bearbeitungsschritte für das Einsetzen und Verformen der Nieten ein sehr aufwendiges Herstellungsverfahren.

Der Erfindung liegt daher die Aufgabe zugrunde, die genannten Nachteile zu vermeiden und eine Biopsie-Zange zu schaffen, deren Herstellung und Montage dadurch vereinfacht ist, daß sie aus wenigen Einzelteilen von einfacher Herstellungsart besteht.

Diese Aufgabe wird erfindungsgemäß dadurch gelöst, daß die zur gelenkigen Verbindung der rückwärtigen Arme der Zangenlöffel mit den Schwenkgliedern dienenden Gelenkachsen einerseits, und die zur Verbindung der Schwenkglieder mit der Seilhülse dienenden weiteren Gelenkachsen andererseits als Achsstummel ausgebildet sind, welche Achsstummel jeweils einstückig mit einem der beiden gelenkig miteinander verbundenen Teile der Biopsie-Zange ausgebildet sind. Die zur Verbindung der rückwärtigen Löffelarme mit den Schwenkgliedern dienenden Achsstummel können dabei jeweils einstückig mit den Schwenkgliedern ausgebildet sein und in Löcher in die rückwärtigen Arme der Zangenlöffel eingreifen, während die zur Verbindung der Schwenkglieder mit der Seilhülse dienenden Achsstummel an zwei einander gegenüber liegenden Seiten dieser Seilhülse angeordnet und einstückig mit dieser ausgebildet sein können. Die Herstellung der Achsstummel an den sie tragenden Gliedern der Biopsiezange kann durch Stanzen oder Prägen erfolgen.

In der Zeichnung ist ein besonders vorteilhaftes Ausführungsbeispiel der Erfindung dargestellt, das im folgenden näher beschrieben wird.

Es zeigen:
- Fig. 1: eine Seitenansicht dieses Ausführungsbeispiels;

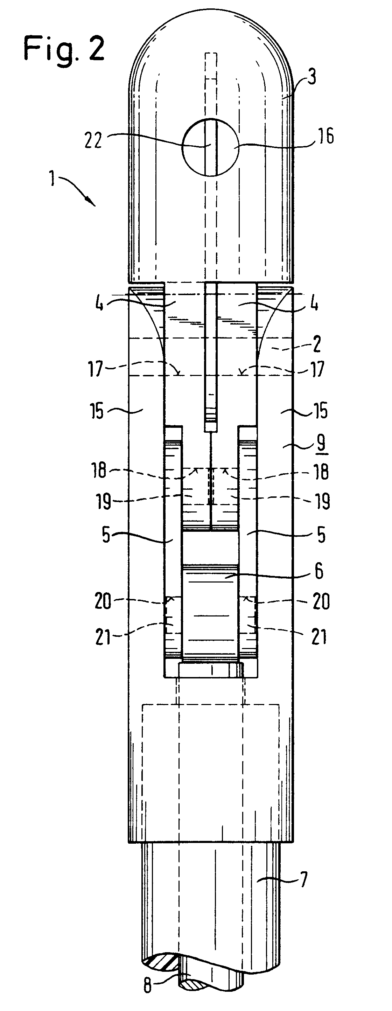

- Fig. 2: eine Seitenansicht des Zangenkopfes dieses Ausführungsbeispiels bei geschlossener Zange;

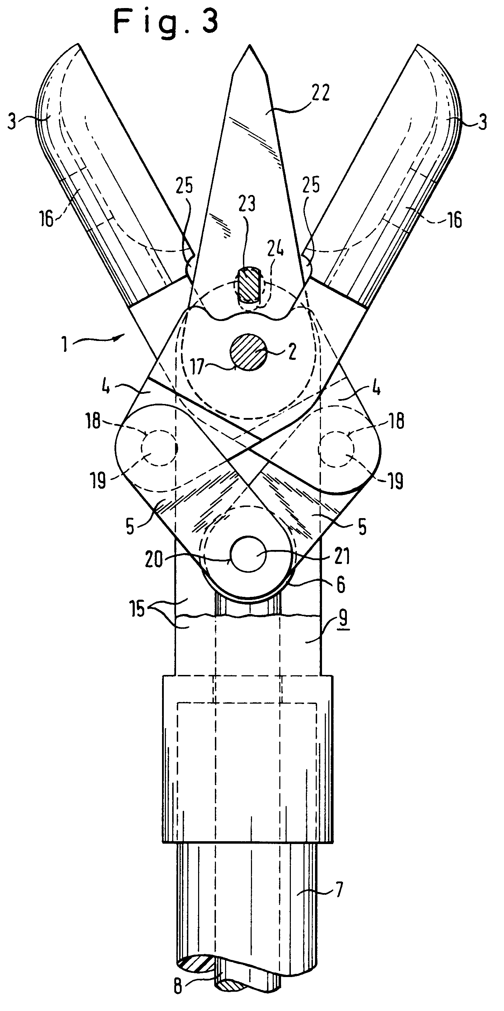

- Fig. 3: eine dazu senkrechte Seitenansicht des Zangenkopfes mit teilweise aufgebrochenem Gehäuse bei geöffneter Zange;

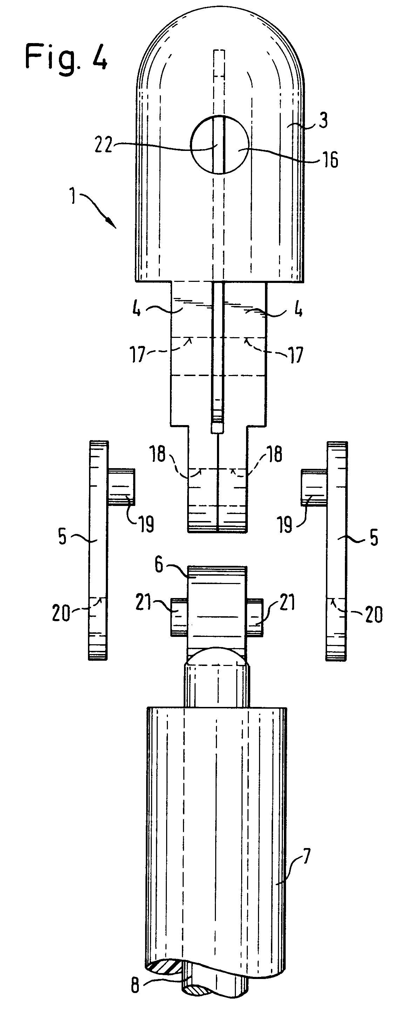

- Fig. 4: eine explosionsartige Darstellung von Einzelteilen des Zangenkopfes dieses Ausführungsbeispiels.

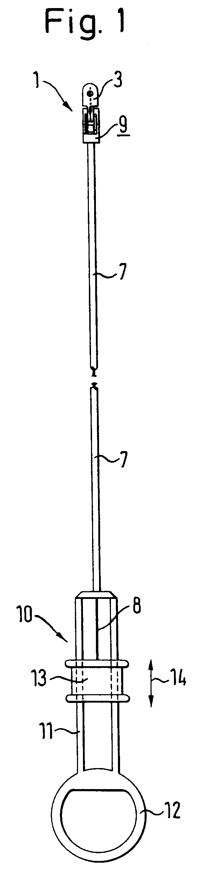

Die in der Zeichnung dargestellte Biopsie-Zange kann z.B. in der Medizin als Vorrichtung zur Entnahme von Gewebsproben eingesetzt werden. Sie hat in ihrem Zangenkopf 1 zwei an einer Zangenachse 2 zueinander schwenkbar gelagerte Zangenlöffel 3, die mit ihren den Löffeln abgewandten rückwärtigen Armen 4 schwenkbar mit dem einen Ende von laschenförmigen Schwenkgliedern 5 verbunden sind. Diese Schwenkglieder sind mit ihrem jeweiligen anderen Ende an einer Seilhülse 6 angelenkt, die auf das vordere Ende eines in einem flexiben Mantel 7 hin- und herbewegbaren Seiles 8 aufgepreßt ist. Am vorderen Ende dieses Mantels ist ein Gehäuse 9 befestigt, in dem eine zur Lagerung der Zangenlöffel dienende Zangenachse 2 angeordnet ist. Am hinteren Ende des Mantels 7 sitzt ein Griff 10, der an seinem vorderen, an den Mantel 7 angeschlossenen Teil 11 rahmenartig ausgebildet ist und an seinem vom Mantel abgewandten Ende einen Griffring 12 aufweist. In den rahmenartigen Griffteil 11 ragt das hintere Ende des Seiles 8 hinein, welches an einen Schieber 13 angeschlossen ist, der an dem rahmenartigen Teil 11 in Richtung des Pfeils 14 in Fig. 1 auf- und abbewegbar ist. Durch diese Schieberbetätigung kann die Biopsie-Zange geöffnet und geschlossen werden.

In das die Zangenlöffel 3 tragende Gehäuse 9 ragt das Seil 8 mit seiner Seilhülse 6 hinein. Das Gehäuse hat zwei Seitenwände 15, an welchen die Zangenachse 2 gelagert ist. Die Zangenlöffel 3 sind bauchig ausgehöhlt und haben in ihrer Mitte jeweils eine Bohrung 16, um die von der Zange erfaßten Gewebeproben o.dgl. leichter aus der Zange entnehmen zu können. Sie sitzen mit einer an ihren rückwärtigen Armen 4 befindlichen Bohrung 17 auf der Zangenachse 2 und haben hinter dieser Bohrung 17 jeweils eine weitere Bohrung 18, in welche die Schwenkglieder mit an derem einem Ende ausgebildeten Achsstummeln 19 eingreifen. Diese Schwenkglieder 5 sind ebenfalls zwischen den Gehäuseseitenwänden 15 angeordnet und haben an ihrem anderen Ende je eine Bohrung 20, in welche Achsstummel 21 eingreifen, die an den den Schwenkgliedern 5 zugewandten Seiten der Seilhülse 6 angeordnet sind, um diese schwenkbar mit den Schwenkgliedern 5 zu verbinden und die Hin- und Herbewegung des Seiles 8 über die Schwenkglieder 5 auf die Zangenlöffel 3 zu übertragen.

Zwischen den Zangenlöffeln und deren rückwärtigen Armen 4 ist ein Dorn 22 eingesetzt, der mit einer Bohrung auf der Zangenachse 2 sitzt. Dieser Dorn hat eine scharfe Spitze, mit der es möglich ist, die Biopsie-Zange zu fixieren, um Gewebe oder zu entnehmende Gewebeproben besser aus dem Gewebe entnehmen zu können. In seiner Ausrichtung gegenüber dem Gehäuse 9 wird der Dorn durch einen Fixierstift 23 gehalten, der eine weitere Bohrung im Dorn durchsetzt und an seinen abgeflachten Enden in Aussparungen 24 im Gehäuse 9 sitzt. Im Bereich dieses Fixierstiftes sind die Löffel 3 sowie ihre rückwärtigen Arme 4 mit Ausnehmungen 25 versehen, um nicht in Kollision mit dem Fixierstift zu gelangen.
- 1: Zangenkopf
- 2: Zangenachse
- 3: Zangenlöffel
- 4: Arm des Zangenlöffels
- 5: Schwenkglied
- 6: Seilhülse
- 7: Mantel
- 8: Seil
- 9: Gehäuse
- 10: Griff
- 11: rahmenartig ausgebildetes Griffteil
- 12: Griffring
- 13: Schieber
- 14: Bewegungsrichtung des Schiebers
- 15: Seitenwand des Gehäuses
- 16: Bohrung im Zangenlöffel
- 17: 1. Achsbohrung im Arm des Zangenlöffels
- 18: 2. Achsbohrung im Arm des Zangenlöffels
- 19: Achsstummel des Schwenkglieds
- 20: Bohrung im Schwenkglied
- 21: Achsstummel der Seilhülse

## Patentansprüche

1. Biopsie-Zange mit zwei an einer Zangenachse zueinander schwenkbar gelagerten Zangenlöffeln, die mit ihren den Löffeln abgewandten rückwärtigen Armen gelenkig mit dem einen Ende von laschenförmigen Schwenkgliedern verbunden sind, welche mit ihrem jeweiligen anderen Ende an einer Seilhülse angelenkt sind, die an dem vorderen Ende eines in einem Mantel hin- und herbewegbaren Seiles sitzt, wobei an dem vorderen Ende des Mantels die Zangenachse gelagert ist,
dadurch **gekennzeichnet,** daß die zur gelenkigen Verbindung der rückwärtigen Arme der Zangenlöffel (3) mit den Schwenkgliedern (5) dienenden Gelenkachsen (19) einerseits und die zur Verbindung der Schwenkglieder (5) mit der Seilhülse (6) dienenden weiteren Gelenkachsen (21) andererseits als Achsstummel (19,21) ausgebildet sind, welche Achsstummel jeweils einstückig mit einem der beiden gelenkig miteinander verbundenen Teile der Biopsie-Zange ausgebildet sind.

2. Biopsie-Zange nach Anspruch 1, dadurch gekennzeichnet, daß die zur Verbindung der rückwärtigen Löffelarme mit den Schwenkgliedern dienenden Achsstummel (19) jeweils einstückig mit den Schwenkgliedern (5) ausgebildet sind und in Löcher in den rückwärtigen Armen der Zangenlöffel (3) eingreifen.

3. Biopsie-Zange nach Anspruch 1, dadurch gekennzeichnet, daß die zur Verbindung der Schwenkglieder (5) mit der Seilhülse (6) dienenden Achsstummel (21) an zwei einander gegenüberliegenden Seiten dieser Seilhülse angeordnet und einstückig mit dieser ausgebildet sind.

4. Biopsie-Zange nach Anspruch 2, dadurch gekennzeichnet, daß die Achsstummel (19) an einem Ende der Schwenkglieder (5) durch Stanzen oder Prägen ausgebildet sind.

5. Biospie-Zange nach Anspruch 3, dadurch gekennzeichnet, daß die Achsstummel (21) an der Seilhülse (6) durch Stanzen oder Prägen ausgebildet sind.

6. Biopsie-Zange nach Anspruch 1, dadurch gekennzeichnet, daß die Zangenlöffel (3) durch Stanzen oder Prägen hergestellt sind.

7. Biopsie-Zange nach den Ansprüchen 4, 5 oder 6, dadurch gekennzeichnet, daß die Schwenkglieder (5), die Seilhülse (6) und/oder die Zangenlöffel (3) aus prägbarem Metall, z.B. Silber oder einer Silberlegierung, bestehen.

8. Biopsie-Zange nach Anspruch 1, dadurch gekennzeichnet, daß auf der Zangenachse (2) zwischen den rückwärtigen Armen (4) der Zangenlöffel (3) ein Dorn (22) sitzt, der in seiner Ausrichtung gegenüber dem Gehäuse (9) durch einen Fixierstift (23) gehalten ist, der eine Bohrung des Dorns durchsetzt und am Gehäuse befestigt ist.
